(19) Europäisches Patentamt
European Patent Office
Office européen des brevets

(11) **EP 4 148 390 A1**

(12) **EUROPEAN PATENT APPLICATION**

(43) Date of publication:
**15.03.2023 Bulletin 2023/11**

(21) Application number: **22183147.2**

(22) Date of filing: **05.07.2022**

(51) International Patent Classification (IPC):
***G01C 21/34*** *(2006.01)*

(52) Cooperative Patent Classification (CPC):
**G01C 21/3469; G01C 21/3415**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2021 JP 2021149765**

(71) Applicant: **TOYOTA JIDOSHA KABUSHIKI KAISHA**
**Toyota-shi, Aichi-ken, 471-8571 (JP)**

(72) Inventors:
• **SHIMADA, Masanori**
**Toyota-shi, 471-8571 (JP)**
• **UEDA, Koichi**
**Toyota-shi, 471-8571 (JP)**
• **SUZUKI, Naoto**
**Toyota-shi, 471-8571 (JP)**
• **SAKAYANAGI, Yoshihiro**
**Toyota-shi, 471-8571 (JP)**

(74) Representative: **D Young & Co LLP**
**120 Holborn**
**London EC1N 2DY (GB)**

(54) **CO2 EMISSION AMOUNT CALCULATION**

(57) A $CO_2$ emission amount calculation apparatus includes a processor, in which the processor searches for a route from a departure place of a movable object to a destination, calculates, in response to re-routing of the movable object on the route searched for, a first $CO_2$ emission amount that the movable object emits in a route before the re-routing and a second $CO_2$ emission amount that the movable object emits in a route after the re-routing, and calculates, with the first $CO_2$ emission amount and the second $CO_2$ emission amount, a $CO_2$ emission amount that the movable object emits over an entire route from the departure place to the destination.

FIG.1

**EP 4 148 390 A1**

**Description**

BACKGROUND OF THE INVENTION

1. Field of the Invention

The present disclosure relates to a $CO_2$ emission amount calculation apparatus and a $CO_2$ emission amount calculation program.

2. Description of the Related Art

Patent Document 1 discloses a technology in which the $CO_2$ emission amount to be emitted from the internal combustion engine of a vehicle in each section from the departure place to the destination is calculated for searching for the $CO_2$ minimization route in which the total $CO_2$ emission amount from the departure place to the destination is minimum.

PRIOR-ART DOCUMENT

PATENT DOCUMENT

Patent Document 1: Japanese Laid-open Patent Publication No. 2010-127690
In calculation of the $CO_2$ emission amount to be emitted in a route from the departure place to the destination, a route change (re-routing) may occur, for example, due to a traffic jam or the choice of a wrong road. Thus, traveling is not necessarily performed along the first expected route. Thus, required has been a technology enabling proper calculation of the $CO_2$ emission amount from the departure place to the destination even in a case where re-routing has occurred.
The present disclosure has been made in consideration of the above. An object of the present disclosure is to provide a $CO_2$ emission amount calculation apparatus and a $CO_2$ emission amount calculation program that enable proper calculation of the $CO_2$ emission amount from the departure place to the destination even in a case where re-routing has occurred.

MEANS FOR SOLVING THE PROBLEM

A $CO_2$ emission amount calculation apparatus according to the present disclosure includes a processor configured to search for a route from a departure place of a movable object to a destination, calculate, in response to re-routing of the movable object on the route searched for, a first $CO_2$ emission amount that the movable object emits in a route before the re-routing and a second $CO_2$ emission amount that the movable object emits in a route after the re-routing, and calculate, with the first $CO_2$ emission amount and the second $CO_2$ emission amount, a $CO_2$ emission amount that the movable object emits over an entire route from the departure place to the destination.
A $CO_2$ emission amount calculation program according to the present disclosure causes a processor to: search for a route from a departure place of a movable object to a destination; calculate, in response to re-routing of the movable object on the route searched for, a first $CO_2$ emission amount that the movable object emits in a route before the re-routing and a second $CO_2$ emission amount that the movable object emits in a route after the re-routing; and calculate, with the first $CO_2$ emission amount and the second $CO_2$ emission amount, a $CO_2$ emission amount that the movable object emits over an entire route from the departure place to the destination.
According to the present disclosure, even in a case where re-routing has occurred, the $CO_2$ emission amount from the departure place to the destination can be properly calculated.

BRIEF DESCRIPTION OF THE DRAWINGS

FIG. 1 is a block diagram of the configuration of a $CO_2$ emission amount calculation apparatus according to an embodiment; and
FIG. 2 is a flowchart of an exemplary processing procedure of a $CO_2$ emission amount calculation method to be performed by the $CO_2$ emission amount calculation apparatus according to the embodiment.

DETAILED DESCRIPTION OF THE PREFERRED EMBODIMENTS

A $CO_2$ emission amount calculation apparatus and a $CO_2$ emission amount calculation program according to an embodiment of the present disclosure will be described with reference to the drawings. Note that the constituent elements in the following embodiment include constituent elements easily replaceable by those skilled in the art and substantially the same constituent elements.

$CO_2$ emission amount calculation apparatus

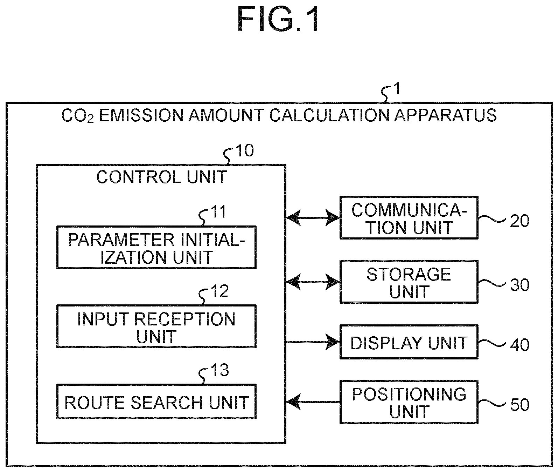

The $CO_2$ emission amount calculation apparatus according to the embodiment will be described with reference to FIG. 1. The $CO_2$ emission amount calculation apparatus is used in order to calculate the $CO_2$ emission amount when a movable object travels from the departure place to the destination.

A movable object to which the $CO_2$ emission amount calculation apparatus according to the embodiment is applied is, for example, a vehicle that travels along a set route. Examples of such a vehicle include an engine vehicle, a hybrid electric vehicle (HEV), a plug-in hybrid electric vehicle (PHEV), a fuel cell electric vehicle (FCEV), and a battery electric vehicle (BEV).

As illustrated in FIG. 1, a $CO_2$ emission amount calculation apparatus 1 includes a control unit 10, a communication unit 20, a storage unit 30, a display unit 40, and a positioning unit 50. Note that the $CO_2$ emission amount calculation apparatus 1 may be mounted on a movable object, such as a vehicle, or a server apparatus capable of communicating with a movable object. In the present embodiment, the $CO_2$ emission amount calculation apparatus 1 mounted on a movable object will be described.

Specifically, the control unit 10 includes a processor, such as a central processing unit (CPU), a digital signal processor (DSP), a field-programmable gate array (FPGA), or a graphics processing unit (GPU), and a memory (main storage unit) including a random access memory (RAM) and a read only memory (ROM).

The control unit 10 executes various types of programs to have centralized control over the operations of various types of constituent elements mounted on the $CO_2$ emission amount calculation apparatus 1. Due to execution of various types of programs, the control unit 10 functions as a parameter initialization unit 11, an input reception unit 12, and a route search unit 13.

The parameter initialization unit 11 initializes and sets various types of parameters for calculation of a $CO_2$ emission amount by the route search unit 13, to be described below. For example, parameters to be initialized and set by the parameter initialization unit 11 are as follows.

The weight M of the movable object [kg]
Travel resistance function Frl(V) [N]
$CO_2$ conversion factor $R_{fuel}$ [kg/kg]
Efficiency function $\eta_{eng}$
External power consumption Et
Gravitational acceleration g [m/s$^2$]

Here, the travel resistance function Frl(V) corresponds to a vehicle speed function and can be given, for example, by the following Expression (1) including coefficients a, b, and c.

$$Frl(V) = a \times V^2 + b \times V + c \qquad (1)$$

The external power consumption corresponds to power consumption, for example, due to the auxiliary machinery or air conditioner of the movable object. As the external power consumption, a previously set fixed value or the current value is used. Note that, in a case where a change in power consumption in the future can be predicted, for example, with a learning model, the predicted power consumption may be used.

As the efficiency function $\eta_{eng}$, a function for the power E[i] that the movable object needs to travel or a fixed value of 1 or less is used.

The input reception unit 12 receives a route search condition as input from a user (e.g., the driver). Examples of such a route search condition include the current date and time, the departure place, the destination, the presence or absence of priority on distance, the presence or absence of priority on arrival time, and the presence or absence of priority on $CO_2$ emission amount.

The input reception unit 12 displays, on the display unit 40, for example, a tab for selection of the current date and time, a tab or box for input of the departure place, a tab or box for input of the destination, a distance priority button, an arrival-time priority button, and a $CO_2$-emission-amount priority button. Then, on the basis of a selection by the user (e.g., a touch operation), the input reception unit 12 receives input. Note that the current date and time may be automatically input on the basis of a clock mounted on the movable object or time information acquired from a global positioning system (GPS) satellite. The departure place may be automatically input on the basis of positional information on the movable object acquired from a GPS satellite.

The route search unit 13 searches for a route from the departure place of the movable object to the destination, on the basis of any route search condition received as input by the input reception unit 12. The route search unit 13 displays, on the display unit 40, for example, a route search start button. Then, on the basis of a selection by the user (e.g., a touch operation), the route search unit 13 starts route search processing. Note that the route search unit 13 may search for one route or a plurality of routes (route candidates) from the set departure place to the set destination.

In response to re-routing of the movable object on the route already searched for, the route search unit 13 re-searches for a route from the current position of the movable object to the destination. Note that the "re-routing" means that the movable object deviates from the route previously searched for and set (makes a change in route), for example, by reason of a traffic jam or the choice of a wrong road. Specifically, the route search unit 13 re-searches for a route from the current position of the movable object acquired by the positioning unit 50 to the destination input at the time of the first route searching. The route search unit 13 may re-search for one route or a plurality of routes (route candidates) from the current position of the movable object to the destination.

In the route search processing of the route search unit 13, for example, Dijkstra's algorithm is used. In the route search processing with Dijkstra's algorithm, the i-th road searched for from the starting point (departure place) to the end point (destination) is subjected to route searching, for example, with the following information.

Travel time T[i] [s]
Road gradient Grad[i] [m/m]
Road length L[i] [m]

Here, the travel time T[i] may vary, depending on the date and time, even on the same road. Thus, as given by the following Expressions (2) and (3), the average vehicle speed V[i] [m/s] and the power E[i] [kW] required in traveling can be calculated per road. Note that, in a case where the movable object (e.g., a vehicle) performs no regeneration, the following Expression (3) satisfies $E[i] \geq 0$.

$$V[i] = L[i]/T[i] \tag{2}$$

$$E[i] = \{(V[i]^2 - V[i-1]^2)/2 + M \times g \times Grad[i] \times L[i] + Frl(V[i]) \times L[i]\}/T[i] + Et[i] \tag{3}$$

The route search unit 13 displays, on the display unit 40, routes (route candidates) searched for (re-searched for) as above. Note that the route search unit 13 may store as necessary a route search result (route re-search result) into the storage unit 30.

Here, in addition to the above route search processing, the route search unit 13 performs, per route searched for, calculation processing for the $CO_2$ emission amount that the movable object emits over the entire route from the departure place to the destination. On the basis of the parameters previously initialized and set by the parameter initialization unit 11, the power E[i], and the travel time T[i], the route search unit 13 calculates the fuel consumption Fuel[i] in accordance with the following Expression (4). Note that the fuel consumption Fuel[i] is not the amount of fuel actually consumed by the movable object but just a predicted value.

$$Fuel[i] = \eta_{eng}(E[i]) \times T[i] \tag{4}$$

Next, the route search unit 13 calculates the $CO_2$ emission amount $CO_2[i]$ in accordance with the following Expression (5).

$$CO_2[i] = Fuel[i] \times R_{fuel}CO_2 \qquad (5)$$

Note that the $CO_2$ emission amount $CO_2[i]$ calculated with the above Expression (5) is not a value calculated with account taken of the amount of energy actually consumed during traveling of the movable object (e.g., the fuel consumption) but a predicted value calculated before traveling of the movable object. In the present embodiment, a predicted value for the $CO_2$ emission amount of the movable object calculated in advance before traveling of the movable object as above is defined as the "advance-prediction $CO_2$ emission amount".

In response to re-routing of the movable object on the route already searched for, in addition to route re-search processing, the route search unit 13 performs, per route re-searched for, calculation processing for the advance-prediction $CO_2$ emission amount over the entire route from the departure place to the destination.

In this case, the route search unit 13 calculates a first $CO_2$ emission amount that the movable object emits in the route before re-routing (route searched for first) ($CO_2$ emission amount before re-routing) and a second $CO_2$ emission amount that the movable object emits in the route after re-routing (route re-searched for) ($CO_2$ emission amount after re-routing). Note that the first $CO_2$ emission amount is required to be part of the $CO_2$ emission amount calculated on the basis of the route searched for first. Then, with the first $CO_2$ emission amount and the second $CO_2$ emission amount, namely, with addition of the second $CO_2$ emission amount to the first $CO_2$ emission amount, the route search unit 13 calculates the advance-prediction $CO_2$ emission amount that the movable object emits over the entire route from the departure place to the destination.

In addition to the advance-prediction $CO_2$ emission amount, the route search unit 13 may calculate a later-prediction $CO_2$ emission amount. The "later-prediction $CO_2$ emission amount" is a predicted value for the $CO_2$ emission amount that the movable object emits and is calculated by accumulation of the advance-prediction $CO_2$ emission amount after traveling of the movable object (or during traveling of the movable object) along the route previously searched for. That is, the later-prediction $CO_2$ emission amount is calculated by accumulation of the advance-prediction $CO_2$ emission amount corresponding to the route along which the movable object has already traveled, in the previously calculated advanced-prediction $CO_2$ emission amount.

Here, even in a case where a new route is re-searched for in response to re-routing of the movable object, the route search unit 13 calculates the later-prediction $CO_2$ emission amount by accumulating the advance-prediction $CO_2$ emission amount corresponding to the route along which the movable object has already traveled, in the advance-prediction $CO_2$ emission amount calculated after re-routing.

In addition to the advance-prediction $CO_2$ emission amount and the later-prediction $CO_2$ emission amount, the route search unit 13 may calculate a later-estimation $CO_2$ emission amount. The "later-estimation $CO_2$ emission amount" is an estimated value for the $CO_2$ emission amount that the movable object emits and is calculated on the basis of the amount of energy actually consumed during traveling of the movable object (e.g., the fuel consumption) after traveling of the movable object along the route previously searched for.

For example, in a case where the movable object is a vehicle including only an internal combustion engine as the power source, an electronic control unit (ECU) in the vehicle detects the actual fuel consumption per unit of time [kg/s]. Then, the route search unit 13 calculates the later-estimation $CO_2$ emission amount with addition of the actual fuel consumption multiplied by the $CO_2$ conversion factor $R_{fuel}$ [kg/kg]. Thus, as the $CO_2$ emission amount that the movable object emits, used can be the later-estimation $CO_2$ emission amount higher in accuracy than the later-prediction $CO_2$ emission amount.

In addition to the routes (route candidates) searched for (re-searched for), the route search unit 13 displays, on the display unit 40, at least one of the advance-prediction $CO_2$ emission amount, the later-prediction $CO_2$ emission amount, and the later-estimation $CO_2$ emission amount calculated as above. Note that the route search unit 13 may store as necessary the advance-prediction $CO_2$ emission amount, the later-prediction $CO_2$ emission amount, and the later-estimation $CO_2$ emission amount into the storage unit 30.

Here, for example, as described in (1) to (3) below, the route search unit 13 may display, on the display unit 40, a result of comparison between any two of the advance-prediction $CO_2$ emission amount, the later-prediction $CO_2$ emission amount, and the later-estimation $CO_2$ emission amount.

(1) Display of Advance-Prediction $CO_2$ Emission Amount and Later-Prediction $CO_2$ Emission Amount on Display Unit 40

Display of the advance-prediction $CO_2$ emission amount and the later-prediction $CO_2$ emission amount on the display unit 40 enables comparison between the $CO_2$ emission amount to the destination expected at the departure place and the $CO_2$ emission amount expected on the basis of the route along which the movable object has actually traveled. This enables mainly visual quantification of variations in $CO_2$ emission amount due to re-routing.

(2) Display of Advance-Prediction $CO_2$ Emission Amount and Later-Estimation $CO_2$ Emission Amount on Display Unit 40

Display of the advance-prediction $CO_2$ emission amount and the later-estimation $CO_2$ emission amount on the display unit 40 enables comparison between the $CO_2$ emission amount to the destination expected at the departure place and the $CO_2$ emission amount estimated to have been actually emitted from the movable object in the route along which the movable object has actually traveled. This enables mainly visual quantification of variations in $CO_2$ emission amount due to re-routing and variations in $CO_2$ emission amount due to the way of running of the movable object (user's driving technique).

(3) Display of Later-Prediction $CO_2$ Emission Amount and Later-Estimation $CO_2$ Emission Amount on Display Unit 40

Display of the later-prediction $CO_2$ emission amount and the later-estimation $CO_2$ emission amount on the display unit 40 enables comparison between the $CO_2$ emission amount expected on the basis of the route along which the movable object has actually traveled and the $CO_2$ emission amount estimated to have been actually emitted from the movable object in the route along which the movable object has actually traveled. This enables mainly visual quantification of variations in $CO_2$ emission amount due to the way of running of the movable object (user's driving technique).

The communication unit 20 includes, for example, a local area network (LAN) interface board and a wireless communication circuit for wireless communication. For example, in a case where the $CO_2$ emission amount calculation apparatus 1 is achieved by a server apparatus capable of communicating with the movable object, the communication unit 20 communicates with the $CO_2$ emission amount calculation apparatus 1, to acquire, for example, a route search result or a result of calculation of a $CO_2$ emission amount from the $CO_2$ emission amount calculation apparatus 1.

The storage unit 30 includes recording media, such as an erasable programmable ROM (EPROM), a hard disk drive (HDD), and a removable medium. Examples of such a removable medium include a universal serial bus (USB) memory and disc recording media, such as a compact disc (CD), a digital versatile disc (DVD), and a Blu-ray (registered trademark) disc (BD). The storage unit 30 can store, for example, an operating system (OS), various types of programs, various types of tables, and various types of databases.

As necessary, the storage unit 30 stores, for example, the various types of parameters set by the parameter initialization unit 11, any route search condition received by the input reception unit 12, a route search result from searching by the route search unit 13, and any $CO_2$ emission amount calculated by the route search unit 13.

The display unit 40 is achieved, for example, by a liquid crystal display (LCD) or an organic EL display (OLED). The display unit 40 may be achieved, for example, by the display of a car navigation system mounted on the movable object. The display unit 40 may be achieved, for example, by a touch panel display having an input function of receiving an operation due to a finger of a user (e.g., the driver) or an operation due to a pen and a display function of displaying various types of information on the basis of the control of the control unit 10.

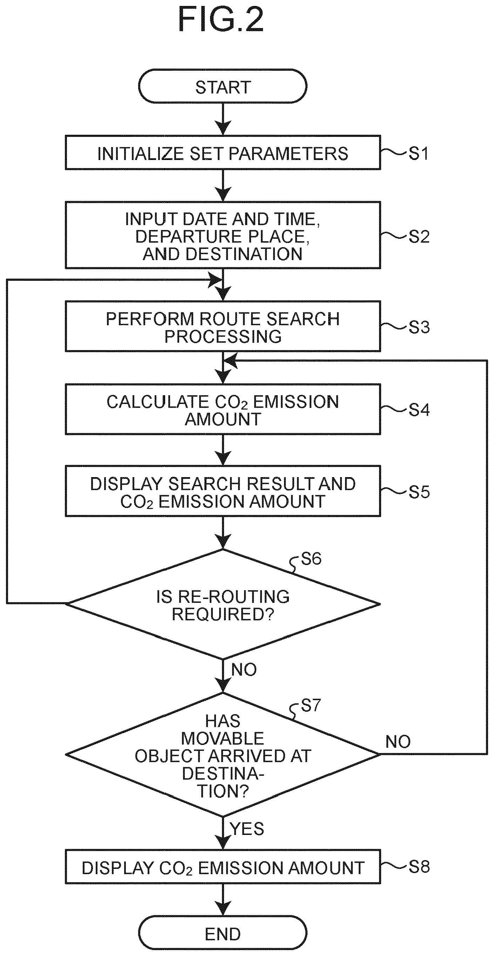

The positioning unit 50 receives radio waves from GPS satellites, to detect positional information and time information on the movable object. Note that the method of detecting positional information on the movable object is not limited to a method with GPS satellites. For example, used may be a method with light detection and ranging or laser imaging detection and ranging (LiDAR) and a 3D map in combination. The positional information on the movable object detected by the positioning unit 50 is used, for example, in route search processing of the route search unit 13 (Step S3 of FIG. 2 to be described below), in determination of whether re-routing is required (Step S6 of FIG. 2), and in determination of whether the movable object has arrived at the destination (Step S7 of FIG. 2).

$CO_2$ Emission Amount Calculation Method

An exemplary processing procedure of a $CO_2$ emission amount calculation method to be performed by the $CO_2$ emission amount calculation apparatus 1 according to the embodiment will be described with reference to FIG. 2.

First, the parameter initialization unit 11 initializes various types of parameters for calculation of a $CO_2$ emission amount (Step S1). Next, the input reception unit 12 receives, as input, route search conditions such as the current date and time, the departure place, and the destination (Step S2).

Next, on the basis of the route search conditions as input received by the input reception unit 12, the route search unit 13 performs route search processing from the departure place of the movable object to the destination (Step S3). Next, the route search unit 13 performs, per route searched for, calculation processing for the $CO_2$ emission amount that the movable object emits (Step S4). Note that, in Step S4, the advance-prediction $CO_2$ emission amount is calculated before traveling of the movable object and the later-prediction $CO_2$ emission amount and the later-estimation $CO_2$ emission amount are calculated during/after traveling of the movable object.

Next, the route search unit 13 displays, on the display unit 40, a route search result and the $CO_2$ emission amount (Step S5). Note that, in Step S5, the route search unit 13 displays, on the display unit 40, the advance-prediction $CO_2$ emission amount.

Next, the route search unit 13 determines whether re-routing is required (Step S6). In Step S6, for example, on the basis of the positional information on the movable object detected by the positioning unit 50, determination of whether re-routing is required is performed. That is, in a case where the current position of the movable object is out of the route searched for in Step S3, it is determined that re-routing is required, otherwise, it is determined that no re-routing is required.

In a case where it is determined in Step S6 that no re-routing is required (No in Step S6), the route search unit 13 determines whether the movable object has arrived at the destination (Step S7). In Step S7, for example, on the basis of the positional information on the movable object detected by the positioning unit 50, determination of whether the movable object has arrived at the destination is performed. That is, in a case where the distance between the current position of the movable object and the destination is a certain value or less, it is determined that the movable object has arrived at the destination, otherwise, it is determined that the movable object has not arrived at the destination.

In a case where it is determined in Step S7 that the movable object has arrived at the destination (Yes in Step S7), the route search unit 13 displays the $CO_2$ emission amount on the display unit 40 (Step S8), and then completes the present processing. Note that, in Step S8, the route search unit 13 displays, on the display unit 40, at least one of the advance-prediction $CO_2$ emission amount, the later-prediction $CO_2$ emission amount, and the later-estimation $CO_2$ emission amount.

Here, in a case where it is determined in Step S6 that re-routing is required (Yes in Step S6), the route search unit 13 goes back to the processing in Step S3. Then, in Step S3, the route search unit 13 re-searches for a route from the current position of the movable object to the destination, followed by the processing of Step S4.

In a case where it is determined in Step S7 that the movable object has not arrived at the destination (No in Step S7), the route search unit 13 goes back to the processing in Step S4 and performs the processing from Step S4.

According to the $CO_2$ emission amount calculation apparatus and the $CO_2$ emission amount calculation program according to the embodiment described above, searching for a route from the departure place to the destination is performed simultaneously with calculation of the $CO_2$ emission amount to be emitted in the route. In response to re-routing on the route searched for, the $CO_2$ emission amount over the entire route from the departure place to the destination is calculated with the $CO_2$ emission amount in the route before re-routing and the $CO_2$ emission amount in the route after re-routing.

Therefore, even in a case where re-routing has occurred, the $CO_2$ emission amount calculation apparatus and the $CO_2$ emission amount calculation program according to the embodiment enable proper calculation of the $CO_2$ emission amount from the departure place to the destination.

According to the $CO_2$ emission amount calculation apparatus and the $CO_2$ emission amount calculation program according to the embodiment, calculation and presentation of the advance-prediction $CO_2$ emission amount and the later-prediction $CO_2$ emission amount enable a user (e.g., the driver) to grasp the $CO_2$ emission amount to be emitted in the travel route and to grasp how much reduction can be made in $CO_2$ emission amount, for example, depending on other route search conditions or re-routing.

According to the $CO_2$ emission amount calculation apparatus and the $CO_2$ emission amount calculation program according to the embodiment, calculation of the later-estimation $CO_2$ emission amount and presentation of the later-estimation $CO_2$ emission amount together with the advance-prediction $CO_2$ emission amount or the later-prediction $CO_2$ emission amount enable a user (e.g., the driver) to grasp how much reduction can be made in $CO_2$ emission amount depending on the way of user's driving.

Although the disclosure has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

EXPLANATION OF SIGNS

1: $CO_2$ EMISSION AMOUNT CALCULATION APPARATUS
10: CONTROL UNIT
11: PARAMETER INITIALIZATION UNIT
12: INPUT RECEPTION UNIT
13: ROUTE SEARCH UNIT
20: COMMUNICATION UNIT
30: STORAGE UNIT
40: DISPLAY UNIT
50: POSITIONING UNIT

**Claims**

1. A $CO_2$ emission amount calculation apparatus comprising

   a processor configured to
   search for a route from a departure place of a movable object to a destination,
   calculate, in response to re-routing of the movable object on the route searched for, a first $CO_2$ emission amount that the movable object emits in a route before the re-routing and a second $CO_2$ emission amount that the movable object emits in a route after the re-routing, and
   calculate, with the first $CO_2$ emission amount and the second $CO_2$ emission amount, a $CO_2$ emission amount that the movable object emits over an entire route from the departure place to the destination.

2. The $CO_2$ emission amount calculation apparatus according to claim 1, further comprising

   a display, wherein
   the processor is configured to display, on the display, the route searched for and the $CO_2$ emission amount that the movable object emits over the entire route from the departure place to the destination.

3. The $CO_2$ emission amount calculation apparatus according to claim 2, wherein

   the processor is further configured to
   calculate, before traveling of the movable object along the route, an advance-prediction $CO_2$ emission amount, based on a plurality of parameters regarding the movable object, the plurality of parameters being previously set,
   calculate, after traveling of the movable object along the route, a later-prediction $CO_2$ emission amount by accumulating the advance-prediction $CO_2$ emission amount,
   calculate, after traveling of the movable object along the route, a later-estimation $CO_2$ emission amount, based on an amount of energy consumed during traveling of the movable object, and
   display, on the display, at least one of the advance-prediction $CO_2$ emission amount, the later-prediction $CO_2$ emission amount, and the later-estimation $CO_2$ emission amount.

4. A $CO_2$ emission amount calculation program for causing a processor to:

   search for a route from a departure place of a movable object to a destination;
   calculate, in response to re-routing of the movable object on the route searched for, a first $CO_2$ emission amount that the movable object emits in a route before the re-routing and a second $CO_2$ emission amount that the movable object emits in a route after the re-routing; and
   calculate, with the first $CO_2$ emission amount and the second $CO_2$ emission amount, a $CO_2$ emission amount that the movable object emits over an entire route from the departure place to the destination.

5. The $CO_2$ emission amount calculation program according to claim 4, further causing the processor to display, on a display included in a $CO_2$ emission amount calculation apparatus, the route searched for and the $CO_2$ emission amount that the movable object emits over the entire route from the departure place to the destination.

6. The $CO_2$ emission amount calculation program according to claim 5, further causing the processor to:

   calculate, before traveling of the movable object along the route, an advance-prediction $CO_2$ emission amount, based on a plurality of parameters regarding the movable object, the plurality of parameters being previously set;
   calculate, after traveling of the movable object along the route, a later-prediction $CO_2$ emission amount by accumulating the advance-prediction $CO_2$ emission amount;
   calculate, after traveling of the movable object along the route, a later-estimation $CO_2$ emission amount, based on an amount of energy consumed during traveling of the movable object; and
   display, on the display, at least one of the advance-prediction $CO_2$ emission amount, the later-prediction $CO_2$ emission amount, and the later-estimation $CO_2$ emission amount.

# FIG.1

# FIG.2

```
            ┌──────────────┐
            │    START     │
            └──────┬───────┘
                   ▼
        ┌─────────────────────────┐
        │ INITIALIZE SET PARAMETERS │──S1
        └────────────┬────────────┘
                     ▼
        ┌─────────────────────────┐
        │   INPUT DATE AND TIME,    │
        │    DEPARTURE PLACE,       │──S2
        │    AND DESTINATION        │
        └────────────┬────────────┘
                     ▼
        ┌─────────────────────────┐
        │   PERFORM ROUTE SEARCH    │──S3
        │       PROCESSING          │
        └────────────┬────────────┘
                     ▼
        ┌─────────────────────────┐
        │   CALCULATE CO2 EMISSION  │──S4
        │         AMOUNT            │
        └────────────┬────────────┘
                     ▼
        ┌─────────────────────────┐
        │  DISPLAY SEARCH RESULT AND │──S5
        │    CO2 EMISSION AMOUNT     │
        └────────────┬────────────┘
                     ▼
```

S6

IS RE-ROUTING REQUIRED?

NO

S7

HAS MOVABLE OBJECT ARRIVED AT DESTINATION?

NO

YES

DISPLAY CO2 EMISSION AMOUNT ──S8

END

Europäisches
Patentamt

European
Patent Office

Office européen
des brevets

## EUROPEAN SEARCH REPORT

Application Number

EP 22 18 3147

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP 2010 127690 A (DENSO CORP) 10 June 2010 (2010-06-10) * abstract; figure 3 * | 1-6 | INV. G01C21/34 |
| X | WO 2011/135661 A1 (PIONEER CORP [JP]; AMANO HIROSHI [JP] ET AL.) 3 November 2011 (2011-11-03) * abstract; figures 5,6 * | 1-6 | |

TECHNICAL FIELDS
SEARCHED      (IPC)

G01C

The present search report has been drawn up for all claims

| Place of search | Date of completion of the search | Examiner |
|---|---|---|
| The Hague | 20 December 2022 | Tabellion, Marc |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.....................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 18 3147

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2022

| Patent document cited in search report | | Publication date | Patent family member(s) | | Publication date |
|---|---|---|---|---|---|
| JP 2010127690 A | | 10-06-2010 | JP | 5180037 B2 | 10-04-2013 |
| | | | JP | 2010127690 A | 10-06-2010 |
| WO 2011135661 A1 | | 03-11-2011 | JP | 5417530 B2 | 19-02-2014 |
| | | | JP WO2011135661 A1 | | 18-07-2013 |
| | | | WO | 2011135661 A1 | 03-11-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010127690 A